# EUROPEAN PATENT SPECIFICATION

(11) **EP 2 645 971 B1**
(45) Date of publication and mention of the grant of the patent: **17.12.2014**
(21) Application number: 11788162.3
(22) Date of filing: 28.11.2011
(51) Int. Cl.: A61F 9/00

(54) **MEDICATED MODULE FOR AN OPHTHALMIC DRUG DELIVERY DEVICE**
MEDIZINISCHES MODUL FÜR EINE VERABREICHUNGSVORRICHTUNG FÜR OPHTHALMISCHE ARZNEIMITTEL
MODULE MÉDICINAL POUR UN DISPOSITIF D'ADMINISTRATION DE MÉDICAMENTS OPHTALMIQUES

(30) Priority: 29.11.2010 EP 10192979
(43) Date of publication of application: 09.10.2013
(73) Proprietor: Sanofi-Aventis Deutschland GmbH, 65929 Frankfurt am Main (DE)
(72) Inventor: KOUYOUMJIAN, Garen, Leamington Spa Warwickshire CV31 1HN (GB); BOYD, Malcolm Stanley, Wellsbourne Warwickshire CV35 9PW (GB)
(74) Representative: Keil & Schaafhausen Patent- und Rechtsanwälte
(86) International application number: PCT/EP2011/071122
(87) International publication number: WO 2012/072546

(56) References cited:
- EP-A1- 0 437 953
- WO-A1-2010/018118
- US-A- 3 016 898
- US-A- 4 981 479
- US-A1- 2005 107 755
- US-A1- 2010 286 633

## Description

### Field of the Present Patent Application

The present application relates to medical devices for delivering at least two drug agents from separate reservoirs using ophthalmic drug delivery devices having only a single activation mechanism, button or trigger and a single dispense interface. A delivery procedure initiated by the user causes a non-user settable dose (i.e., a fixed dose) of a second drug agent along with a set dose of a first drug agent to be delivered to the patient. The drug agents may be available in two or more reservoirs, containers, or packages, each containing independent (single drug compound) or pre-mixed (co-formulated multiple drug compounds) drug agents.

Document US 2005/0107755 A1 discloses a system for delivering a single drop of liquid to an eye of a subject comprising a holder which enables the user to retract the lower lid and dispense a drop of a liquid with a one-hand operation. Further, the holder comprises two rod members whose lower ends are fixed to a capillary tube at which upper end a loading chamber is secured. The loading chamber is connected to a deformable air-filled bulb which projects beyond bent portions of the holder so that a portion of the bulb is compressed by the fingers to dispense the liquid. The loading chamber further incorporates flap valve which defines the upper end of the loading chamber and another flap valve which defines the lower end of the loading chamber. In addition, there is a cover valve into which a nozzle of a dispenser can be introduced in order to fill the loading chamber with liquid. For liquid dispense, the user presses the flexible bulb with the forefinger to cause a drop of the liquid contained in the loading chamber to pass from the loading chamber into the capillary tube to the outlet thereof to be dispensed into the cul-de-sac of the lower lid of the eye.

The document US 2010/0286633 A1 discloses a precision lid retracting eyedropper device that includes means to hold the user's eyelid in an open position during the application of the eye drops. The precision eyedropper comprises a solution holding bladder located in the top portion of a housing. Further, a pump assembly comprising an inwardly directed check valve and an outwardly directed check valve and a resilient tube is located in the central portion of the housing. Each time a push button is pushed down causing a deformation of a resilient pump tube forcing a precise amount of solution out of check valve and out of exit tip. When the push button is released by the user, the resulting suction caused by tube returning to its original position forces a new amount of solution into incoming check valve. The bladder may be refilled using the opening accessible by removing cap wherein the cap is replaced when bladder is full.

From document EP 0 437 953 A1 an apparatus for applying a medicament to an eye is known with a housing constructed and arranged to retain a dropper bottle. To utilize the known apparatus front opening end of the housing is placed over the eye with an engagement body resting against lower eyelid. Thereby, an elongated drive shaft is displaced so that a projection passes through housing slot to compress the dropper bottle and force drops of liquid medicament from a nozzle.

Document US 3,016,898 refers to a fluid applicator for human eyes comprising a fluid deposited within a chamber from a conventional container. When an eye cup is positioned over the eye a button is actuated to open a valve and close a valve whereby a controlled definite size drop of fluid is passed downwardly through a passage defined by the sleeve to drop upon the cornea of the eye.

Devices containing two types of medicaments are known from the documents US-A-4981479 and WO-A-2010/018118.

### Background

Certain disease states require treatment using one or more different medicaments. Some drug compounds need to be delivered in a specific relationship with each other in order to deliver the optimum therapeutic dose. This invention is of particular benefit where combination therapy is desirable, but not possible in a single formulation for reasons such as, but not limited to, stability, compromised therapeutic performance, and/or toxicology.

A number of potential problems can arise when delivering two active medicaments or "agents" simultaneously. As just one example, the two active agents when present in a single container may interact with each other during the long-term, shelf life storage of the formulation. Therefore, there are certain advantages to storing the active components separately and then potentially combine them at the point of delivery, e.g. injection, needle-less injection, pumps, or inhalation. However, any potential process for combining the two or more agents needs to be straightforward and convenient for the user to perform reliably, repeatedly, and safely.

One further concern is that the quantities and/or proportions of each active agent making up the potential combination dose or therapy may need to be varied for each user or at different stages of their therapy. Again, as just one example, one or more active agents may require a titration period to gradually introduce a patient to a "maintenance" dose. A further example would be if one active agent requires a non-adjustable fixed dose while the other agent is varied in response to a patient's symptoms or physical condition. This potential concern could mean that pre-mixed formulations of multiple active agents may not be suitable as these pre-mixed formulations would have a fixed ratio of the active components, which could not be varied by the healthcare professional or user.

Additional concerns may arise where a multi-drug compound therapy is required, because certain users may not be able to cope with having to use more than one drug delivery system or make the necessary accurate calculation of the required dose combination. This is especially true for users with dexterity or computational difficulties.

Accordingly, there exists a strong need to provide devices for the delivery of two or more medicaments in a single activation of an ophthalmic drug delivery device, such as an eyedropper, that is simple and safe for the user to perform and that also tends to reduce a patient's anxiety towards taking repeated doses of medicaments.

### SUMMARY

The present application discloses a medicated module attachable to an ophthalmic drug delivery device, preferably an eyedropper containing multiple doses of a primary or first medicament. Most preferably the eyedropper would be a squeeze bottle. The medicated module of this invention provides a means by which the user is able to receive doses of two medicaments from a single activation of the primary device thus reducing the issues with regard to storage and complexity of operation. Maintaining the method of operation used to actuate the primary device alone is believed to ensure good comparability between medicament delivery of the first medicament from the primary device when used in isolation, as well as when it is combined with the medicated module.

The module itself can include features that complement or seek to improve the design or functionality of the primary device. Such features could include an eye cup to aid in the correct application, a spray nozzle to improve drug distribution over drops or a valve mechanism for dose volume selection / control. In the preferred embodiment shown, the medicated module is affixed to the primary bottle using the thread already present on such bottles for retaining the bottle cap, but alternative mounting solutions are possible, such as an enclosure that the primary device is placed within or a clip affixed to the bottle itself. Such attachment means could reasonably be developed to provide exclusive attachment between the primary bottle and secondary module, thereby preventing accidental or intentional misuse with other drugs or non-approved medicated modules.

Prior to use, it is likely that the medicated module would be packed separately to the primary dropper bottle. The module would feature a means of maintaining the integrity of the secondary medicament prior to use such as foil seals over apertures that may be removed by the user prior to use or automatically by the primary device during fitting or actuation. In addition, the module would likely use valves (such as simple silicone dome valves) to retain the secondary medicament within the module drug cavity or reservoir until dispensed by the user.

In one possible embodiment of the invention, the secondary drug is dispensed through the shunting action of the primary drug being forced through the valves in the medicated module when the primary bottle is squeezed to release the primary or first medicament. The valves in the medicated module prevent backflow of the mixture of drugs into the primary ophthalmic drug delivery device, preferably a squeeze bottle. The inherent resistance of the valves to the hydraulic pressure that builds up in the device by squeezing the bottle can also be used to ensure that the primary drug is only released through the medicated module once sufficient pressure has been built up in the system to ensure the entirety of the secondary drug is flushed out and administered to the user along with the dose of the primary medicament. In an alternative embodiment, this pressure could be used in conjunction with the aforementioned spray nozzle to convert the device from a dropper delivery system to a spray delivery system, which may be advantageous due to the potential for better coverage and drug retention afforded by a spray.

The use of the medicated module allows for possibility to have a suite of different medicated modules for use in the manner described, in order to potentially treat various conditions, or to provide a range of titration options. In addition, although in the embodiment shown the secondary module contains a single secondary drug, it is possible to include more than one drug cavity or reservoir in series for the dispense of several secondary drugs, or to design the secondary module itself such that a multiplicity of modules can be mounted to the primary device.

The system may also be configured to help ensure that the medicated module is only capable of being used once by a user. This may be achieved through integration of a mechanical flap (or similar flow restriction means) that is only activated (closed off) following flushing of the secondary drug. Alternatively, the valves used to control flow of drug through the secondary drug cavity could be designed to activate only once and subsequently block fluid flow.

Prior to use, i.e., attachment to an ophthalmic dropper, the medicated module provides a means of containing the second medicament within a sealed drug cavity or reservoir. Potential materials that might be used to form the drug cavity that holds the second medicament might include (but are not limited to); Acetal (polyoxymethylene, polyacetal or polyformaldehyde), COC (Cyclo Olefin Copolymer), COP (Cyclo Olefin Polymer) and PBT (Polybutylene Terephthalate). Sealing of the reservoir in the medicated module may be achieved through a combination of foil seals (single or multilayer), stopper bungs, septa or like means known in the art.

On assembly to the primary device, the reservoir in the medicated module is automatically engaged thus establishing the flow path to the outlet orifice at the distal end of the medicated module. Preferably, connections in the flow path could be established using a compliant material, such as rubber or TPE, to help ensure minimal or no leakage occurs during dispense and thus delivered dose performance is maintained. The act of engaging the fluid path of the reservoir with that of the first medicament in the ophthalmic device automatically during assembly simplifies the users interaction with the device.

According to the present invention a medicated module is for use with an ophthalmic drug delivery device. The medicated module comprises a housing configured for removable attachment to a discharge opening of an ophthalmic drug delivery device. The housing has a distal end and a proximal end. A reservoir contained within the housing containing a dose of medicament, where the reservoir is positioned between two valves. Activation of an ophthalmic drug delivery (2) device causes the valves to open allowing discharge of the medicament through an orifice in the distal end.

In one embodiment of the invention the medicated module for use with an ophthalmic drug delivery device includes a housing configured for removable attachment to the cap fitting of an ophthalmic dropper container, such as a squeeze bottle, where the housing has a distal end and a proximal end. There is at least one reservoir contained within the housing having at least one dose of second medicament, where the reservoir is positioned between a first and second pressure valve. When the module is attached to the ophthalmic dropper device, activation of the dropper device creates fluid pressure that opens the normally closed valves allowing the medicament contained in the reservoir to be dispense through the distal end of the module and into the eye.

In a further embodiment, the medicated module may have one or more seals on either side of the pressure valves to ensure the sterility of the medicament before use and also to maintain the interior chamber of the medicated module sterile until immediately prior to use.

Although the reservoir of the medicated module can be designed in a number of configurations and orientations, one preferred configuration is inline with the axis of the orifice of the ophthalmic device. In some instances it might be desirable to include a slider or other movable piece on the medicated module such that the user can remove one or more internal seals associated with the reservoir. Additionally, it may be desirable to include an eyecup or similar alignment device at the distal end of the medicated module to assist the user in accurate delivery of the first and second medicaments or to adjust the flow path of the drug so as to make it more ergonomic for the user, for example so that they do not need to tilt their head backwards to apply drops.

In a preferred embodiment a master drug compound (i.e., a first medicament) is contained within a multiple dose eye drop squeeze container or bottle that is used with a single use, user-replaceable, module that contains a single dose of a secondary medicament and a single dispense interface or orifice. When connected to the drug delivery device the secondary compound is activated/delivered on dispense of the primary compound. Any number of drugs or drug combinations, such as analgesics, hormones, beta agonists or corticosteroids, or a combination of any of the above-mentioned drugs could be used with the invention.

Examples of beta agonists are, without limitation, salbutamol, levosalbutamol, terbutaline, pirbuterol, procaterol, metaproterenol, fenoterol, bitolterol mesylate, salmeterol, formoterol, bambuterol, clenbuterol, indacaterol.

Hormones are for example hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

These as well as other advantages of various aspects of the present invention will become apparent to those of ordinary skill in the art by reading the following detailed description, with appropriate reference to the accompanying drawings.

### BRIEF DESCRIPTION OF THE DRAWINGS

Exemplary embodiments are described herein with reference to the drawings, in which:
Figure 1 illustrates a perspective view of one possible ophthalmic drug delivery device for use with the medicated modules of the present invention.
Figure 2 illustrates a medicated module according to the present invention attached to the ophthalmic device shown in Fig. 1; and
Figure 3 illustrates a sectional view of one arrangement of a medicated module attached to the ophthalmic device shown in Fig. 1.

### DETAILED DESCRIPTION

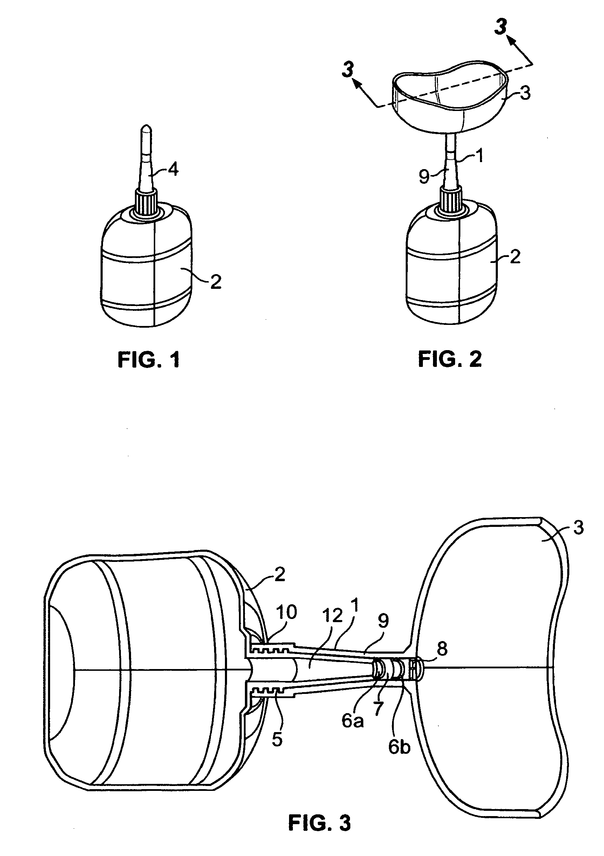

The presently proposed medicated module may be used with any and all designs of ophthalmic drug delivery devices, for example the eyedropper shown in Fig. 1 that uses a squeeze bottle to discharge one or more drops of a first medicament. In one arrangement, the presently proposed medicated module 1 administers one or more single doses of a second medicament simultaneously with a variable dose of a first medicament from a multi-dose squeeze container through a single output or drug dispense interface such as module eyepiece 3. A single activation of container 2, for example by squeezing the sides of the container inwardly, will cause both the first and second medicaments to be delivered to the user. Preferably, the volume and size of the dose of the second medicament is independently controlled by the design and manufacture of the reservoir 7 in the medicated module and therefore not influenced by the size of the dose generated by the primary ophthalmic delivery device during activation. This fixed dose of the second medicament contained within the medicated module may be a single dose.

In a preferred arrangement, the drug dispense interface comprises a module dispense interface, such as the eyecup 3 shown in Figs. 2 and 3, however, any orifice or discharge opening capable of passing drops, a liquid stream or spray could be used. As shown in Fig.1, the primary device 2 has a covering cap 4 fitted to the container by threads 5 (see Fig. 3). The medicated module 1 has a proximal end 10 that is configured to be removably attached to the same connection means, in this case, threads. The housing 9 can be attached to the primary device 2 using any reversible connection means known to the art, for example, threads, snap locks, snap fits, detents, luer locks, bayonet, snap rings, keyed slots, and combinations of such connections. Preferably, the attachment mechanism between the medicated module and the ophthalmic device is configured such that the user can easily attach the module to the primary device and then easily remove the module after expelling the second medicament contained in the module. In certain applications, the connector or attachment configuration may comprise an exclusive attachment where such an exclusive attachment would only allow such a medicated module to be attached to certain types of primary drug delivery devices and prevented from being attached to other types of ophthalmic devices or other drug delivery devices. Once the module is removed, the primary device can then be used as a stand-alone drug delivery device to administer the first or primary medicament.

Referring now to Fig. 3, this figure shows a close-up of a cross-sectional view of the medicated module 1 attached to primary device 2. Reservoir 7 is inside housing 9 and is configured hold the second medicament. Preferably, the reservoir is contained between pressure valves 6a and 6b positioned on either side of the reservoir. The pressure valves can also act as seals to prevent contamination of the medicament and may be designed in such a way as to only release and allow fluid flow through the medicated module when sufficient pressure has been built up in the primary device to guarantee flushing of the full dose of secondary medicament from the module.

The user operates the combination of medicated module 1 and dropper 2 in exactly the same manner as they would if the module was not attached. The user would place the distal end of the module or eyecup 3 over one eye and squeeze container 2 to force out a dose of the primary medicament into the discharge channel 12 of container 2. As used herein, "distal end" refers to end of the device where the medicament is dispensed. This activation of container 2 also creates a fluid pressure in channel 12 and inside the medicated module at the proximal side of the first pressure valve 6a causing this valve to open, pressurizing the reservoir and forcing the second medicament and primary medicament through valve 6b and out of the distal end of the module. Preferably the valves 6a and 6b are configured as one-way check valves to prevent backflow in the proximal direction and possibly into container 2. In a preferred embodiment, a nozzle 8 is included at the outlet of the channel of housing 9 immediately before eyecup 3. The nozzle could be configured to convert an otherwise droplet(s) of combined medicaments into a spray or aerosol. Depending on the design of the reservoir and /or the properties of the medicaments, the first medicament may be expelled with the second medicament as a mixture or may be expelled sequentially.

In one preferred arrangement, the reservoir comprises a single dose of the second medicament. Alternatively, the reservoir comprises a single dose of a premix of active agents or medicaments. In one preferred arrangement, this primary medicament comprises a different type of medicament as the medicament contained within the drug delivery device.

It is within the scope of the invention to configure the medicated module with a locking mechanism so as to lock and/or block the distal end, proximal end, or both after dose administration such as with a collapsing medicament chamber or mechanical single-use valves. One advantage of locking the medicated module from repeated use is that a user will be prevented from reusing an expended medicated module and therefore eliminate the possibility that a user would use the expended medicated module under the assumption that he or she is receiving the predefined dose of the primary medicament stored in a new medicated module. Likewise, such a blocking/locking feature prevents a user from re-using a non-sterile medicated module after a dose has been delivered.

The medicated module arrangements herein disclosed are preferably self-contained and may be provided as a sealed and sterile disposable module. Although not shown, the medicated modules disclosed herein could be supplied by a manufacturer contained in a protective and sterile capsule or container where the user would peel or rip open a seal or the container itself to gain access to the sterile medicated module or that could be punctured or opened automatically during the action of fitting or actuating the medicated module. In some instances it might be desirable to provide two or more seals for each end of the medicated module.

Moreover, in the arrangements discussed above, these arrangements have the benefit in that the second medicament is contained entirely within the medicated module, separate and away from the first medicament contained within the ophthalmic drug delivery device.

Exemplary embodiments of the present invention have been described. Those skilled in the art will understand, however, that changes and modifications may be made to these embodiments without departing from the true scope of the present invention, which is defined by the claims.

### List of References

- 1: medicated module
- 2: container
- 3: module eyepiece
- 4: covering cap
- 5: threads
- 6a: pressure valves
- 6b: pressure valves
- 7: reservoir
- 8: nozzle
- 9: housing
- 10: proximal end
- 12: channel

## Claims

1. A medicated module for use with an ophthalmic drug delivery device comprising multiple doses of a primary medicament in a container, the medicated module **characterized by**:
a housing (9) configured for removable attachment to a discharge opening of an ophthalmic drug delivery device (2), where the housing has a distal end and a proximal end; and
a reservoir (7) contained within the housing containing one or more single doses of a secondary medicament, where the reservoir is positioned between two valves (6a, 6b);
wherein activation of an ophthalmic drug delivery (2) device causes the valves to open allowing discharge of a variable dose of the primary medicament simultaneously with the one or more single doses of the secondary medicament through an orifice (8) in the distal end.

2. The medicated module of claim 1 further comprising an eyecup (3) at the distal end.

3. The medicated module of claim 1 or 2, wherein operation of the ophthalmic eyedropper (2) comprising squeezing the sides of the container inwardly.

4. The medicated module of any preceding claim further comprising a spray nozzle (8) to deliver the medicament to an eye.

5. The medicated module of any preceding claim where the secondary medicament is a single dose of a liquid.

6. The medicated module of claim 1 or 2 further comprising seals located on either side of the valves (6a, 6b).

7. The medicated module of claim 1 further comprising an exclusive attachment configuration where such an exclusive attachment allows the medicated module to be attached to certain types of ophthalmic drug delivery devices only and prevents from being attached to other types of ophthalmic drug delivery devices.

8. The medicated module of claim 1 wherein the valves (6a, 6b) are one-way check valves.

9. The medicated module of claim 1 further comprising a locking mechanism so as to lock and/or block its distal end, proximal end, or both after dose administration, such as with a collapsing medicament chamber or mechanical single-use valves, preventing a user from reusing the expended medicated module.

10. An ophthalmic drug delivery system comprising, in combination,
a medicated module (1) of any of claims 1 to 9; and
a squeeze bottle (2) comprising the container with the multiple doses of the primary medicament.

## Patentansprüche

1. Medizinisches Modul zur Verwendung mit einer Verabreichungsvorrichtung für ophthalmische Arzneimittel, die mehrere Dosierungen eines primären Medikaments in einem Behälter enthält, wobei das medizinische Modul **gekennzeichnet ist durch** ein Gehäuse (9) zur lösbaren Befestigung an einer Abgabeöffnung einer Verabreichungsvorrichtung (2) für ophthalmische Arzneimittel, wobei das Gehäuse ein distales Ende und eines proximales Ende aufweist; und einen Aufnahmebehälter (7) in dem Gehäuse, der eine oder mehrere Einzeldosen eines sekundären Medikaments enthält, wobei der Aufnahmebehälter zwischen zwei Ventilen (6a, 6b) angeordnet ist;
worin Aktivierung einer Verabreichungsvorrichtung (2) für ophthalmische Arzneimittel Öffnen der Ventile verursacht, so dass eine variable Dosis des primären Medikaments gleichzeitig mit der einen oder den mehreren Einzeldosen des sekundären Medikaments **durch** eine Öffnung (8) in dem distalen Ende abgegeben werden kann.

2. Medizinisches Modul nach Anspruch 1, ferner eine Augenwanne (3) an dem distalen Ende umfassend.

3. Medizinisches Modul nach Anspruch 1 oder 2, worin die Bedienung des ophthalmischen Augentropfers (2) das Zusammendrücken der Seiten des Behälters nach innen umfasst.

4. Medizinisches Modul nach einem der vorhergehenden Ansprüche, ferner eine Sprühdüse (8) zur Abgabe des Medikaments an ein Auge umfassend.

5. Medizinisches Modul nach einem der vorhergehenden Ansprüche, worin das sekundäre Medikament eine Einzeldosis einer Flüssigkeit ist.

6. Medizinisches Modul nach Anspruch 1 oder 2, ferner Dichtungen umfassend, die auf beiden Seiten der Ventile (6a, 6b) angeordnet sind.

7. Medizinisches Modul nach Anspruch 1, ferner eine exklusive Befestigungskonfiguration umfassend, wobei eine solche exklusive Befestigung die Befestigung des medizinischen Moduls nur an bestimmten Arten von Verabreichungsvorrichtungen für ophthalmische Arzneimittel gestattet und die Befestigung an anderen Arten von Verabreichungsvorrichtungen für ophthalmische Arzneimittel verhindert.

8. Medizinisches Modul nach Anspruch 1, worin die Ventile (6a, 6b) Einweg-Absperrventile sind.

9. Medizinisches Modul nach Anspruch 1, ferner einen Verriegelungsmechanismus umfassend zum Verriegeln und/oder Blockieren seines distalen Endes, seines proximalen Endes oder von beiden nach Verabreichung der Dosis, beispielsweise mit einer kollabierenden Medikamentenkammer oder mechanischen Einmalventilen, wodurch verhindert wird, dass ein Anwender das verbrauchte medizinische Modul erneut verwendet.

10. Verabreichungssystem für ophthalmische Arzneimittel, in Kombination umfassend
ein medizinisches Modul (1) nach einem der Ansprüche 1 bis 9; und
eine Quetschflasche (2), die den Behälter mit den mehreren Dosierungen des primären Medikaments umfasst.

## Revendications

1. Module contenant une substance médicamenteuse destiné à être utilisé avec un dispositif de distribution de médicament ophtalmique comprenant de multiples doses d'une substance médicamenteuse principale dans un réceptacle, le module contenant une substance médicamenteuse étant **caractérisé par** un logement (9) configuré en vue d'être fixé de manière amovible à une ouverture de sortie d'un dispositif de distribution de médicament ophtalmique (2), le logement comportant une extrémité distale et une extrémité proximale ; et un réservoir (7) contenu à l'intérieur du logement et contenant une ou plusieurs doses individuelles d'une substance médicamenteuse secondaire, le réservoir étant placé entre deux soupapes (6a, 6b) ;
l'activation d'un dispositif de distribution de médicament ophtalmique (2) entraînant l'ouverture des soupapes, ce qui permet à une dose variable de la substance médicamenteuse principale de sortir en même temps que la ou les doses individuelles de la substance médicamenteuse secondaire par un orifice (8) dans l'extrémité distale.

2. Module contenant une substance médicamenteuse selon la revendication 1, comprenant en outre une oeillère (3) située à l'extrémité distale.

3. Module contenant une substance médicamenteuse selon la revendication 1 ou 2, l'actionnement du compte-gouttes oculaire (2) comprenant l'application d'une pression sur les côtés du réceptacle vers l'intérieur.

4. Module contenant une substance médicamenteuse selon l'une quelconque des revendications précédentes, comprenant en outre une buse de pulvérisation (8) pour distribuer la substance médicamenteuse à un oeil.

5. Module contenant une substance médicamenteuse selon l'une quelconque des revendications précédentes, la substance médicamenteuse secondaire étant une dose individuelle d'un liquide.

6. Module contenant une substance médicamenteuse selon la revendication 1 ou 2, comprenant en outre des joints se trouvant de chaque côté des soupapes (6a, 6b).

7. Module contenant une substance médicamenteuse selon la revendication 1, comprenant en outre une configuration de fixation exclusive, ladite fixation exclusive permettant la fixation du module contenant une substance médicamenteuse uniquement à certains types de dispositifs de distribution de médicament ophtalmique et empêchant sa fixation à d'autres types de dispositifs de distribution de médicament ophtalmique.

8. Module contenant une substance médicamenteuse selon la revendication 1, les soupapes (6a, 6b) étant des soupapes de non-retour.

9. Module contenant une substance médicamenteuse selon la revendication 1, comprenant en outre un mécanisme de verrouillage pour verrouiller et/ou bloquer son extrémité distale, son extrémité proximale, ou les deux après l'administration d'une dose, par exemple avec un compartiment à substance médicamenteuse compressible ou des soupapes mécaniques à usage unique, empêchant un utilisateur de réutiliser le module contenant une substance médicamenteuse usagé.

10. Système de distribution de médicament ophtalmique comprenant, en combinaison,
un module contenant une substance médicamenteuse (1) selon l'une quelconque des revendications 1 à 9 ; et
un flacon souple (2) comprenant le réceptacle avec les multiples doses de la substance médicamenteuse principale.
